# EUROPEAN PATENT APPLICATION

(11) **EP 4 681 667 A2**
(43) Date of publication of application: **21.01.2026**
(21) Application number: 25190119.5
(22) Date of filing: 17.07.2025
(51) Int. Cl.: A61B 18/02, A61B 90/00, A61B 18/00

(54) **CRYOABLATION PROBES FOR DIRECTIONAL ICE FORMATION**

(30) Priority: 19.07.2024 US 202418778719
(71) Applicant: Varian Medical Systems, Inc., Palo Alto, CA 94304 (US)
(72) Inventor: GAUD, Ishan, Austin, 78717 (US)
(74) Representative: Mathisen & Macara LLP

(57) **Abstract**

A cryoprobe (200) includes a shell (202) extending in an axial direction (212) and defining an inner cavity. The cryoprobe also includes a tip (204) connected to a distal end (210) of the shell and a cryogen supply (206) extending in the inner cavity that is configured to provide a flow of cryogen toward the distal end of the shell. The cryoprobe also includes a directional insulator (208) positioned radially outward of the cryogen supply in the inner cavity. The directional insulator guides the cryogen flow in a direction away from the distal end of the shell along a predetermined return path between the cryogen supply and the shell.

## Description

### FIELD

The present disclosure relates to cryoablation probes and related methods that form ice in a desired direction or that produce asymmetrical ice formations.

### BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase) that may be passed through a probe in thermal contact with the target tissue. Heat from the tissue passes from the tissue, through the probe, and into the cryogen that removes heat from the targeted tissue. This removal of heat causes tissue to freeze, resulting in the destruction of the targeted tissue. It is desirable that the cryogen is of sufficiently low temperature to quickly and efficiently cause the targeted tissues to freeze.

Traditional or existing cryoprobes and related structures often produce ice formations or iceballs that are symmetrical around an axis of the cryoprobe. Such ice formations are often spherical or elliptical in shape. It may be desirable to produce asymmetrical ice formations or to direct the formation of ice in a particular direction from the cryoprobe. There exists a need, therefore, for improved cryoprobes and related methods that may produce asymmetrical ice formation and/or produce ice that is directed in a desired direction from the cryoprobe.

### SUMMARY

This section provides a general summary of the disclosure, and is not a comprehensive disclosure of its full scope or all of its features.

In various embodiments of the present disclosure, a cryoprobe is provided as defined in the appended claims. The cryoprobe may include a shell extending in an axial direction and defining an inner cavity, a tip connected to a distal end of the shell, a cryogen supply extending in the inner cavity and configured to provide a flow of cryogen toward the distal end of the shell, and a directional insulator positioned radially outward of the cryogen supply in the inner cavity. The directional insulator may guide the cryogen flow in a direction away from the distal end of the shell along a predetermined return path between the cryogen supply and the shell. The cryoprobe may include a needle, which is suitably an elongate member. The shell may be a tubular member that is elongated in an axial direction along a longitudinal axis. The shell may be a hollow cylindrical member that forms the needle of the cryoprobe. The components of the cryoprobe are suitably located within the inner cavity. The cryogen supply may be substantially aligned along the longitudinal axis of the shell.

In one aspect, the directional insulator may insulate a portion of the shell from the cryogen flow. The directional insulator may be positioned in the inner cavity radially between the cryogen supply and the shell. Suitably, the directional insulator has a tubular shape with a portion of the tubular wall of the insulator absent. The cryogen return path may be formed of the outside surface of the cryogen supply, the inner surface of the shell, a first axial surface of the directional insulator, and a second axial surface of the directional insulator. The directional insulator may be fixed in the shell at a desired position.

In another aspect, the directional insulator may have an open annulus cross-sectional shape and the open portion of the annulus defines the predetermined return path for the cryogen flow. An angle A may define the portion of the directional insulator that is absent and forms the cryogen return path. The angle A of the directional insulator may be various sizes depending on a desired size and direction of the ice that may form outside the shell at the cryogen return path. In one example, the angle A is about 90 degrees. In other examples, the angle A may have other values such as an angle in the range of about 30 degrees to about 180 degrees.

In another aspect, the cryoprobe may be configured to produce ice that grows in a direction outward from the predetermined return path.

In another aspect, the cryoprobe may be configured to limit growth of ice at locations on the shell other than at the predetermined return path.

In another aspect, the cryoprobe may include a vacuum sleeve positioned inside the inner cavity. The vacuum sleeve may be connected to an end of the directional insulator. The vacuum sleeve may be positioned radially outward of the cryogen supply.

In another aspect, the directional insulator may be configured to rotate about an axis of the shell inside the inner cavity.

In another aspect, the cryoprobe may include one or more indicators positioned on an external surface of the shell, and the one or more indicators may indicate a location of the predetermined return path.

In another aspect, the directional insulator may fill a space between the cryogen supply and the shell except at the predetermined return path.

In some embodiments of the present disclosure, a cryoprobe may include a shell extending in an axial direction and defining an inner cavity. The cryoprobe may also include a tip connected to a distal end of the shell, and a cryogen supply extending in the inner cavity that is configured to provide a flow of cryogen toward the distal end of the shell. The cryoprobe may also include at least one heater positioned on the shell. The shell may be a tubular member that is elongated in an axial direction along a longitudinal axis. The cryoprobe may comprise a return path along which cryogen may flow away from the distal end. The return path for the cryogen may be located between the outer surface of the cryogen supply and the inner surface of the shell.

In one aspect, the at least one heater may be deposited on an external surface of the shell in a conductive ink.

In another aspect, the at least one heater may include at least two different conductive inks each having a different resistance.

In another aspect, the at least one heater may include a plurality of heaters each positioned at a different circumferential location on the external surface of the shell. In another embodiment, each of the heaters may include a different conductive ink, each having a different resistance.

In another aspect, each heater of the plurality of heaters is independently controllable to limit ice formation at desired locations.

In another aspect, the cryoprobe may also include a vacuum sleeve positioned in the inner cavity, wherein an ice formation zone is located at an axial location on the shell between the tip and the vacuum sleeve, and the at least one heater is located on the shell in the ice formation zone.

In some embodiments of the present disclosure a method of directing ice formation on a cryoprobe is provided. The method may include moving cryogen toward a tip of a cryoprobe through a cryogen supply, and limiting formation of ice on a portion of an external surface of the cryoprobe.

In one aspect, the step of limiting formation of ice may include guiding return cryogen through a predetermined return path.

In another aspect, the step of limiting formation of ice may include blocking a flow of return cryogen using a directional insulator positioned radially between the cryogen supply and a shell of the cryoprobe.

In another aspect, the step of limiting formation of ice may include energizing at least one heater positioned on a portion of the cryoprobe where ice formation is not desired.

In another aspect, the at least one heater is formed by depositing conductive ink on the cryoprobe.

In some embodiments of the present disclosure, a cryoablation apparatus as defined in the appended claims. Suitably, the cryoablation apparatus may include a cryoablation console with a cryogen delivery apparatus. The console may be coupled to one of the cryoprobes of the present disclosure that may include an ice limiting device that is configured to limit the growth or formation of ice so that ice may be formed in a predetermined direction relative to the needle of the cryoprobe. The cryoablation console may include a cryo-controller, a cryogen delivery apparatus and a cryogen source. The cryo-controller may be configured to cause the cryogen to be moved through a cryogen flow path that includes a cryogen supply line from the cryogen source through a cryogen line to the cryoprobe. The cryo-controller may be configured to cause the cryogen may then flow back to the cryogen source via a cryogen return line from the cryoprobe back to the cryogen source.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an illustration of an example cryoablation apparatus in accordance with some embodiments of the present disclosure.
FIG. 2 is an isometric view of an example cryoprobe in which the tip and shell are transparent illustrating various aspects of some embodiments of the present disclosure.
FIG. 3 is a cross-sectional view of the example cryoprobe of FIG. 2.
FIG. 4 is an isometric view of another example cryoprobe that may include one or more heaters in accordance with some embodiments of the present disclosure.
FIG. 5 is an isometric view of the cryoprobe of FIG. 4 in which the shell is transparent illustrating various aspects of some embodiments of the present disclosure.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

In various embodiments of the present disclosure, a cryoprobe is provided that is configured to direct the formation of ice in a particular direction. Existing or traditional cryoprobes typically form iceballs or ice formations that are substantially symmetrical about an axis of the cryoprobe. Traditional or existing cryoprobes typically form ice in a spherical shape or elliptical shape about the cryoprobe. In some circumstances such an ice formation may not be desirable. In some circumstances, a target tissue that is desired to be destroyed using cryoablation techniques may have an asymmetrical shape or the target tissue may be located near to a body structure or healthy body tissue. It may be desirable to grow ice in a particular direction from the cryoprobe to avoid damaging healthy body tissues. In still other examples, the target tissue may have a particular shape and it may be desirable to grow ice in a particular direction to destroy the target tissue.

The cryoprobes of the present disclosure may grow ice in a particular direction by limiting the growth of ice in some directions relative to the cryoprobe. In some examples, the cryoprobe may include a directional insulator that may guide or limit the flow of cryogen in the cryoprobe to cause ice to form in a desired direction. In other examples, the cryoprobe may include one or more heaters positioned on the cryoprobe that may be energized to reduce the transfer of heat away from particular tissues. In such a manner, the growth of ice may be limited in a desired direction thus allowing ice to form in another direction. Various examples of such cryoprobes and related methods are further described below.

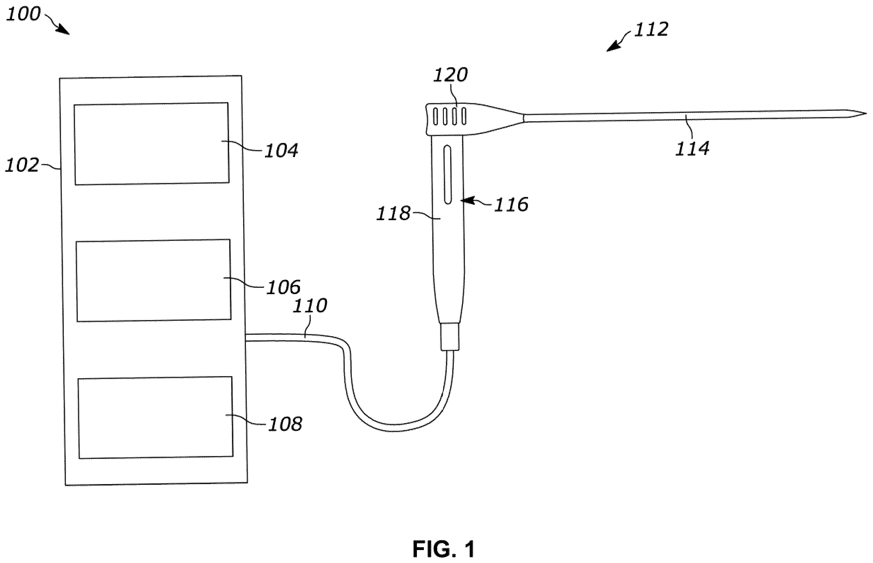

Referring now to FIG. 1, an example cryoablation apparatus 100 is shown. The cryoablation apparatus 100 may include a cryoablation console 102, and a cryoprobe 112. The cryoablation console 102 may include a cryo-controller 104, a cryogen delivery apparatus 106 and a cryogen source 108. The cryo-controller 104 may include a computing device or other controller that can be used to control delivery of a cryogen (e.g., Argon, Helium, Nitrogen, or the like) from the cryogen source 108 to the cryoprobe 112 using the cryogen delivery apparatus 106. The cryogen source 108 may be a suitable Dewar or other container that can be filled with a cryogen. The cryogen delivery apparatus 106 may include a pump, one or more valves, and other suitable fluid delivery devices to fluidly connect the cryogen source to the cryogen line 110 of the cryoprobe 112. Upon the initiation of a freezing cycle, the cryo-controller 104 may cause the cryogen to be moved through a cryogen flow path that includes a cryogen supply line from the cryogen source through the cryogen line 110 to the cryoprobe 112. The cryogen may then flow back to the cryogen source via a cryogen return line from the cryoprobe 112 back to the cryogen source 108.

The cryogen line 110 may be a flexible tube or other conduit that may include multiple lumens to allow the cryogen to flow in a supply direction to the cryoprobe 114 and separately in a return direction away from the cryoprobe 114. The cryogen line 110 may be of sufficient length to allow the console 102 to be positioned near the patient in a treatment room and to allow the patient to be moved into and out of an imaging device. In some examples, the cryogen line may be at least about 3.65 meters (12 feet) in length. In other examples, the cryogen line 110 may have other lengths.

The cryogen line 110 fluidly couples the cryogen source 108 to the cryoprobe 112. The cryoprobe 112 may include a needle 114. The needle 114 may be a pointed cylindrical tool or other elongated member that is configured to be inserted into patient tissue and be positioned at or near the target tissue during treatment. The needle 114 may be configured as a pointed tool having an outer diameter in a range of about 1 mm to about 4 mm. The cryoprobe 112 may also include a handle 116. The handle 116 may be configured with a first (or proximate) portion 118 and a second (or distal) portion 120. The first portion 118 may be substantially aligned with the cryogen line 110 and the second portion 120 may offset at an angle relative to the first portion 118. The offset angle between the first portion 118 and the second portion 119 may be about 90 degrees to define a right angle handle. In other example, the first portion 118 and the second portion 120 may be offset at different angles. In still other examples, the cryoprobe 112 may be substantially linear and the handle 116 may be aligned in a same direction as the needle 114.

In some examples, the handle 116 may include a vacuum chamber positioned at or near an outside surface of the handle 116. The vacuum chamber may insulate the exterior of the handle from the extremely low operating temperatures of the cryogen that moves through the handle to the cryoprobe 112. This may allow an operator to touch or otherwise manipulate the cryoprobe 112 during treatment.

While not shown, more than one cryoprobe 112 may be coupled to the console 102. Multiple cryoprobes 112 may be used during a single cryoablation treatment in combination. The console 102 may be configured to deliver cryogen to each of the multiple cryoprobes 112. The cryoprobes 112 may be similar to each other or may be different to produce iceballs of different sizes and shapes so as to freeze and destroy the target tissue.

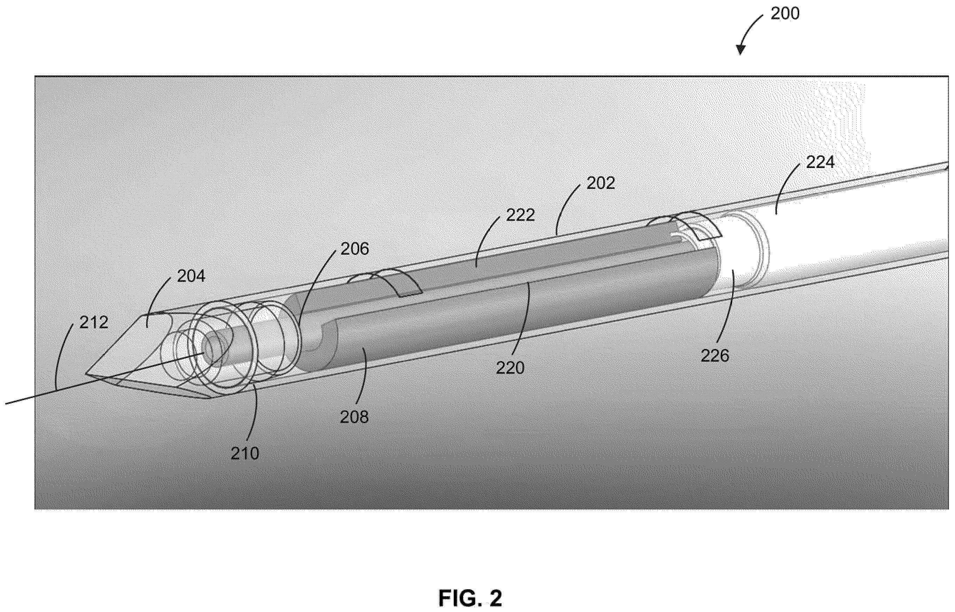

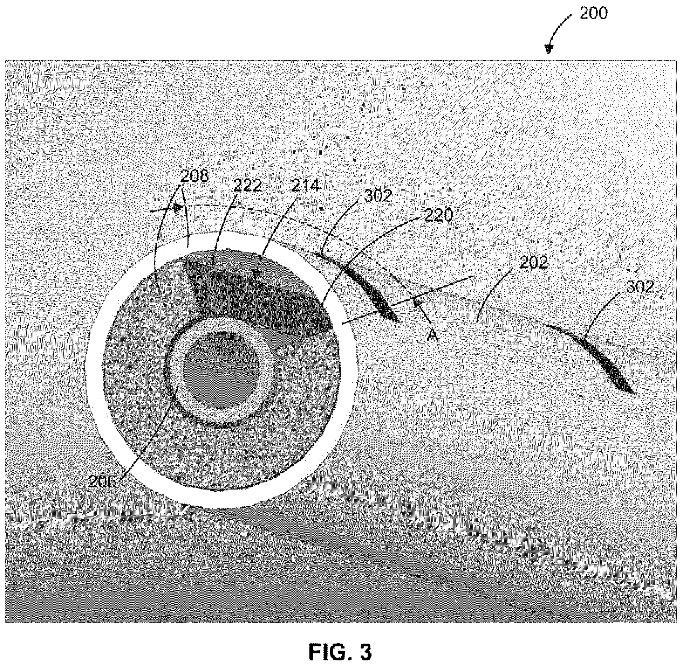

Referring now to FIGs. 2 and 3, an example cryoprobe 200 is shown. The cryoprobe 200 may include a shell 202, a tip 204, a cryogen supply 206, and a directional insulator 208. The shell 202, in FIG. 2, is transparent for illustration purposes to show the internal elements of the cryoprobe 200. The shell 202 may be a cylindrical hollow member that forms the needle of the cryoprobe 200. The shell 202 may be a length of stainless steel or other rigid tubing that may define an inner cavity in which various other components of the cryoprobe 200 are located. A tip 204 may be fixed to a distal end 210 of the shell 202. The shell 202 and the tip 204 may form an enclosed inner cavity in which cryogen may flow during operation. The cryogen may flow through the cryoprobe 200 to remove heat from the location of the target tissue. The cryogen may have a sufficiently low temperature to cause ice to form around the shell 202 during operation.

The cryogen may be transported to the distal end 210 of the shell 202 via the cryogen supply 206. The cryogen supply 206 may be a length of tubing that may be coupled to the cryogen source as previously described. The cryogen supply 206 may be substantially aligned inside the inner cavity of the shell 202 along the axis 212 of the shell 202. During operation, the cryogen may flow toward the distal end 210 of the cryoprobe 200 through the cryogen supply 206. The cryogen may then flow away from the distal end 210 of the shell 202 via a cryogen return path. The cryogen may then be exhausted or returned to the Dewar or other cryogen source.

The directional insulator 208 may be positioned in the inner cavity radially between the cryogen supply 206 and the shell 202. The directional insulator 208 may be positioned in the space that would otherwise form a return path for the cryogen in traditional cryoprobes. The directional insulator 208 may block the flow of cryogen and/or may guide the cryogen to a predetermined cryogen return path 214. In the example shown, the directional insulator 208 has a tubular shape with a portion of the tubular wall of the insulator 208 absent. The return cryogen may flow along the cryogen return path 214 formed from the outside surface of the cryogen supply 206, the inner surface of the shell 202, a first axial surface 220 of directional insulator 208, and a second axial surface 222 of directional insulator 208.

As shown in FIG. 3, a cross-section of the directional insulator 208 shows that the member may have an annular shape with a sector of the annulus removed to allow cryogen to pass inside the shell 202 along a predetermined portion of the inner surface of the shell 202. Since the cryogen flows in the cryogen return path 214 rather than along and/or around the entire inner surface of the shell 202, thermal transfer from the tissue(s) that surrounds the shell 202 is limited to an ice formation zone positioned externally to the cryogen return path 214. The directional insulator 208 limits thermal transfer with the cryogen since the directional insulator 208 occupies the space in the shell 202 other than at the cryogen return path 214.

The directional insulator 208 may be made of a various insulating materials or constructions such as a ceramic material, plastic material, insulating polymer, a vacuum chamber, or the like. The directional insulator 208 may be fixed in the shell 202 at a desired position. In some examples, the cryoprobe 200 may include a vacuum sleeve 224. The vacuum sleeve 224 may be positioned in the inner cavity of the shell radially outward of the cryogen supply 206. The vacuum sleeve 224 may insulate a portion of the shell 202 that is located away from the tip 204. The directional insulator 208 may be connected to a distal end 226 of the vacuum sleeve 224. This may fix the directional insulator 208 at a desired axial position in the shell 202. In other examples, the directional insulator 208 may be fixed in a desired position using other attachments, adhesives, crimps, staking, welding, or the like.

In still other examples, the directional insulator 208 may be movable in the shell 202. The directional insulator 208 may, for example, be allowed to rotate in the shell 202. The user may be able to rotate the directional insulator 208 in the shell 202 to move or position the cryogen return path 214 at a desired circumferential position. The directional insulator 208 may be fixed to the vacuum sleeve 224 and the directional insulator 208 may be rotated with the vacuum sleeve 224 via a dial or lever that may be positioned in the handle (not shown in FIG. 2) of the cryoprobe 200. In other examples, other rotational configurations may be used.

As discussed above, the directional insulator 208 may wrap around a portion of the cryogen supply 206. The directional insulator 208 may define the cryogen return path 214 by a sector of the annulus that is absent from the directional insulator 208. An angle A (see FIG. 3) may define the portion of the directional insulator 208 that forms the cryogen return path 214. The angle A of the directional insulator 208 may be various sizes depending on a desired size and direction of the ice that may form outside the shell 202 at the cryogen return path 214. In one example, the angle A is about 90 degrees. In other examples, the angle A may have other values such as an angle in the range of about 30 degrees to about 180 degrees. In other examples, the angle A may have other values.

As further shown in FIG. 3, the cryoprobe 200 may include one or more indicators 302. The indicators 302 may be markings on the exterior of the shell 202. The indicators 302 may indicate a location of the cryogen return path 214 and indicate to a user where the ice will form during operation. The indicators 302 may also be spaced axially at known intervals to indicate a distance that the cryoprobe 200 has been inserted (or retracted) from a tissue.

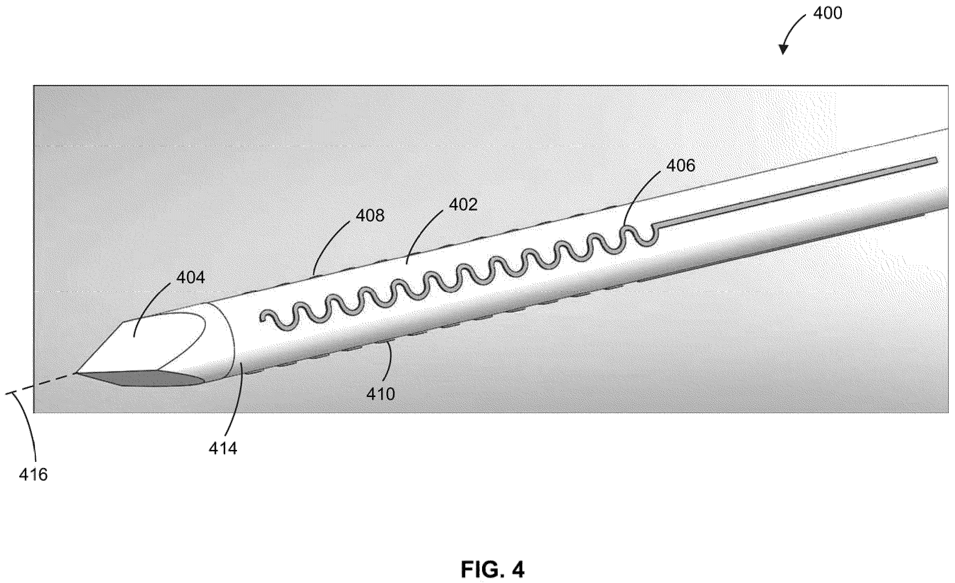

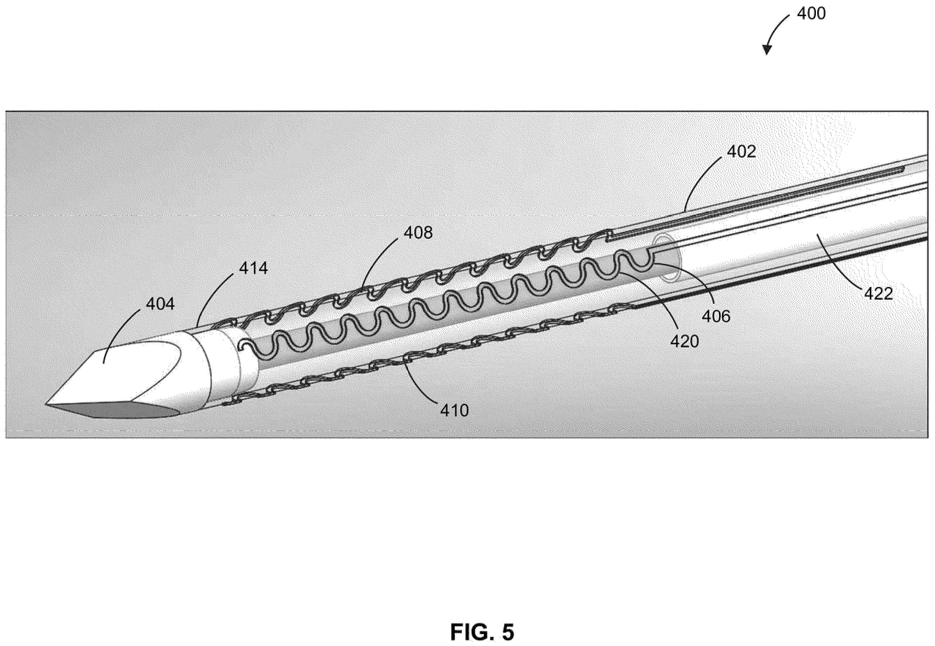

Referring now to FIGs. 4, and 5, another example cryoprobe 400 is shown. In this example, the cryoprobe 400 may include one or more heaters that may be used to limit the formation of ice at one or more locations on the cryoprobe 400. The example cryoprobe 400 may include a shell 402, a tip 404, a first heater 406, a second heater 408, and a third heater 410. The cryoprobe 400 may also include a fourth heater that is located on the back side of the cryoprobe 400 that is not visible in the illustration. The shell 402 may be similar to the shell 202 previously described. The shell 402 may be a tubular member that is elongated in an axial direction along axis 416. The shell 402 may be a stainless steel tube or other rigid material that defines an inner cavity. The tip 404 may be connected to the shell 402 at a distal end 414.

As shown in FIG. 5 in which the shell 202 is shown as transparent for illustration purposes, a cryogen supply 420 and a vacuum sleeve 422 may be positioned in the inner cavity of shell 202. The cryogen supply 420 may provide a flow of cryogen to the distal end 414 of the shell where the cryogen may flow out of the cryogen supply 420 and then follow a return path away from the distal end 414. The return path for the cryogen may be located between the outer surface of the cryogen supply 420 and the inner surface of the shell 402. This flow of cryogen may cool the cryoprobe 400 at an axial location located toward the tip 404.

The heaters of the cryoprobe 400 may be located at the ice formation zone of the cryoprobe 400. The ice formation zone may be located toward the distal end 414 of the cryoprobe at an axial location between the vacuum sleeve 422 and the tip 404. The first heater 406, the second heater 408, and the third heater 410 may be located at this axial position. The first heater 406, the second heater 408, and the third heater 410 may be spaced circumferentially around the shell 402 in the ice formation zone. The heaters may be spaced in various intervals around the circumference. In the example shown, the heaters are spaced at about 90 degree intervals around the circumference of the shell 402. In other examples, other quantities of heaters may be used and/or may be spaced at other intervals around the circumference of the shell 402.

Each of the first heater 406, the second heater 408, and the third heater 410 may be electrically coupled to a power supply. The power supply (not shown) may be located in the ablation console 102 previously described and may be controlled by the cryo-controller 104. The first heater 406, the second heater 408, and the third heater 410 may be independently controlled and independently energized to heat a portion of the shell 402. In this manner, the ice that would normally form in the ice formation zone may be limited due to the heat generated by one or more of the heaters.

The first heater 406, the second heater 408, and the third heater 410 may be positioned on an external surface of the shell 402. The first heater 406, the second heater 408, and/or the third heater 410 may be deposited on surface of the shell 402 using a conductive ink. The conductive ink may be deposited on the surface using a printer head. The first heater 406, the second heater 408, and the third heater 410 may have various shapes. In the example shown, the first heater 406, the second heater 408, and the third heater 410 may have a resistive portion that may have a sinusoidal or series of curved shapes. Such a configuration may allow the heat that is generated by the heater to be distributed across a surface of the shell. The first heater 406, the second heater 408, and the third heater 410 may also have a lead portion that functions as a connective wire to transfer the power signal from the power supply to the resistive portion. The resistive portion of the heater may be located in the ice formation zone and may be made of first type of conductive ink with a known resistance that generates heat when power is supplied. The lead portion of the heater may have a low resistance to efficiently transfer the power signal to the resistive portion. Thus, the resistive portion and the lead portion of the heaters may be made of at least two different conductive inks and/or have different resistances. In other embodiments, a cryoprobe may include other configurations of heaters and other cryoprobes may include more or less heaters than the cryoprobe 400.

In some embodiments of the present disclosure, method of directing ice formation during a cryoablation cycle are provided. In one example, a method of directing ice formation may include providing one or more cryoprobes described herein. The method may include initiating or causing a flow of cryogen to the cryoprobe. Such flow of cryogen may cause thermal energy to be removed from a target tissue that may be located at or near the cryoprobe. The method may also include limiting the formation of ice through the use of a cryoprobe of the present disclosure.

The limitation of ice formation may be accomplished, for example, by blocking the flow of cryogen along one or more portions of the cryoprobe. A directional insulator may be used, for example, to block the flow of return cryogen along a portion of the shell of the cryoprobe. The method may include guiding a flow of cryogen along a portion of the cryoprobe shell at which it is desired to form ice. The cryogen flow may be guided using a directional insulator as previously described. By guiding the flow of cryogen along a portion of the shell, ice may form at that predetermined location on the cryoprobe and ice may be directed to form at this location.

In other embodiments, one or more heaters may be used to limit the growth of ice at one or more positions on the cryoprobe. A heater may be positioned on the shell of the cryoprobe and may be energized after the flow of cryogen is initiated. The heater may limit the transfer of thermal energy and the freezing process (iceball formation) via the cryogen at the locations in which the heater is energized. Thus, ice may be directed to from at locations on the cryoprobe away from a location where a heater is energized. In other examples, combinations of the above methods and aspects of the different cryoprobes described herein may be combined into a single cryoprobe to direct the formation of ice in a predetermined or desired direction.

The following is a list of non-limiting illustrative embodiments disclosed herein:

Illustrative embodiment 1: A cryoprobe comprising: a shell extending in an axial direction and defining an inner cavity; a tip connected to a distal end of the shell; a cryogen supply extending in the inner cavity and configured to provide a flow of cryogen toward the distal end of the shell; and a directional insulator positioned radially outward of the cryogen supply in the inner cavity, the directional insulator guiding the cryogen flow in a direction away from the distal end of the shell along a predetermined return path between the cryogen supply and the shell.

Illustrative embodiment 2: The cryoprobe of illustrative embodiment 1, wherein the directional insulator insulates a portion of the of the shell from the cryogen flow.

Illustrative embodiment 3: The cryoprobe of Illustrative embodiment 1 or 2, wherein the directional insulator comprises an open annulus cross-sectional shape and the open portion of the annulus defines the predetermined return path for the cryogen flow.

Illustrative embodiment 4: The cryoprobe of any of illustrative embodiments 1 to 3, wherein the cryoprobe is configured to produce ice that grows in a direction outward from the predetermined return path.

Illustrative embodiment 5: The cryoprobe of any of illustrative embodiments 1 to 3, wherein the cryoprobe is configured to limit growth of ice at locations on the shell other than at the predetermined return path.

Illustrative embodiment 6: The cryoprobe of any of illustrative embodiments 1 to 5, further comprising a vacuum sleeve positioned inside the inner cavity, the vacuum sleeve connected to an end of the directional insulator.

Illustrative embodiment 7: The cryoprobe of any of illustrative embodiments 1 to 6, wherein the directional insulator is configured to rotate about an axis of the shell inside the inner cavity.

Illustrative embodiment 8: The cryoprobe of any of illustrative embodiments 1 to 7, further comprising one or more indicators positioned on an external surface of the shell, the one or more indicators indicating a location of the predetermined return path.

Illustrative embodiment 9: The cryoprobe of any of illustrative embodiments 1 to 8, wherein the directional insulator fills a space between the cryogen supply and the shell except at the predetermined return path.

Illustrative embodiment 10: A cryoprobe comprising: a shell extending in an axial direction and defining an inner cavity; a tip connected to a distal end of the shell; a cryogen supply extending in the inner cavity and configured to provide a flow of cryogen toward the distal end of the shell; and at least one heater positioned on the shell.

Illustrative embodiment 11: The cryoprobe of illustrative embodiment 10, wherein the at least one heater is deposited on an external surface of the shell in a conductive ink.

Illustrative embodiment 12: The cryoprobe of any of illustrative embodiments 10 or 11, wherein the at least one heater comprises at least two different conductive inks each having a different resistance.

Illustrative embodiment 13: The cryoprobe of any of illustrative embodiments 10 to 12, wherein the at least one heater comprises a plurality of heaters each positioned at a different circumferential location on the external surface of the shell.

Illustrative embodiment 14: The cryoprobe of any of illustrative embodiments 10 to 13, wherein each heater of the plurality of heaters is independently controllable to limit ice formation at desired locations.

Illustrative embodiment 15: The cryoprobe of any of illustrative embodiments 10 to 14, further comprising a vacuum sleeve positioned in the inner cavity, wherein an ice formation zone is located at an axial location on the shell between the tip and the vacuum sleeve, the at least one heater located on the shell in the ice formation zone.

Illustrative embodiment 16. A method of directing ice formation on a cryoprobe comprising: moving cryogen toward a tip of a cryoprobe through a cryogen supply; and limiting formation of ice on a portion of an external surface of the cryoprobe.

Illustrative embodiment 17: The method of illustrative embodiment 16, wherein the step of limiting formation of ice comprises guiding return cryogen through a predetermined return path.

Illustrative embodiment 18: The method of any of illustrative embodiments 16 or 17, wherein the step of limiting formation of ice comprises blocking a flow of return cryogen using a directional insulator positioned radially between the cryogen supply and a shell of the cryoprobe.

Illustrative embodiment 19: The method of any of illustrative embodiments 16 to 18, wherein the step of limiting formation of ice comprises energizing at least one heater positioned on a portion of the cryoprobe where ice formation is not desired.

Illustrative embodiment 20: The method of any of illustrative embodiments 16 to 19, wherein the at least one heater is formed by depositing conductive ink on the cryoprobe.

Illustrative embodiment 21: A cryoablation apparatus comprising: a cryogen delivery apparatus configured to supply a flow of cryogen; and a cryoprobe operably coupled to the cryogen delivery apparatus, the cryoprobe comprising an ice formation limiting device

Illustrative embodiment 22:The cryoablation apparatus of illustrative embodiment 21, wherein the ice formation limiting device comprises a directional insulator positioned radially outward of a cryogen supply in a needle of the cryoprobe.

Illustrative embodiment 23:The cryoablation apparatus of illustrative embodiment 22, wherein the directional insulator comprises an open annulus cross-sectional shape of insulating material and the open portion of the annulus defines a predetermined return path for the cryogen flow.

Illustrative embodiment 24:The cryoablation apparatus of illustrative embodiment 21, wherein the ice formation limiting device comprises a heater positioned on a needle of the cryoprobe.

Illustrative embodiment 25:The cryoablation apparatus of illustrative embodiment 24, wherein the heater is deposited on an external surface of the needle in at least two different conductive inks each having a different resistance.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A cryoprobe (112, 200) comprising:
a shell (2020) extending in an axial direction (212) and defining an inner cavity;
a tip (204) connected to a distal end (210) of the shell;
a cryogen supply (206) extending in the inner cavity and configured to provide a flow of cryogen toward the distal end (210) of the shell; and
a directional insulator (208) positioned radially outward of the cryogen supply (206) in the inner cavity, the directional insulator guiding the cryogen flow in a direction away from the distal end (210) of the shell along a predetermined return path (214) between the cryogen supply (206) and the shell (202).

2. The cryoprobe of claim 1, wherein the directional insulator (208) insulates a portion of the of the shell (202) from the cryogen flow.

3. The cryoprobe of claim 2, wherein the directional insulator comprises an open annulus cross-sectional shape and the open portion of the annulus defines the predetermined return path (214) for the cryogen flow, and, optionally, the directional insulator (208) fills a space between the cryogen supply (206) and the shell (202) except at the predetermined return path (214).

4. The cryoprobe of claim 2 or 3, wherein the cryoprobe is configured to produce ice that grows in a direction outward from the predetermined return path (214) and, optionally, the cryoprobe is configured to limit growth of ice at locations on the shell other than at the predetermined return path (214).

5. The cryoprobe of any one of the preceding claims, further comprising a vacuum sleeve (224) positioned inside the inner cavity, the vacuum sleeve connected to an end of the directional insulator (208).

6. The cryoprobe of any one of the preceding claims, wherein the directional insulator (208) is configured to rotate about an axis (212) of the shell inside the inner cavity.

7. The cryoprobe of any one of the preceding claims, further comprising one or more indicators (302) positioned on an external surface of the shell (202), the one or more indicators indicating a location of the predetermined return path (214).

8. A cryoprobe (112, 400) comprising:
a shell (402) extending in an axial direction and defining an inner cavity;
a tip (404) connected to a distal end (410) of the shell;
a cryogen supply (406) extending in the inner cavity and configured to provide a flow of cryogen toward the distal end (414) of the shell; and
at least one heater (406, 408, 410) positioned on the shell.

9. The cryoprobe of claim 8, wherein the at least one heater (406, 408, 410) is deposited on an external surface of the shell in a conductive ink, and, optionally, the at least one heater (406, 408, 410) comprises at least two different conductive inks each having a different resistance.

10. The cryoprobe of claim 8 or 9, wherein the at least one heater (406, 408, 410) comprises a plurality of heaters each positioned at a different circumferential location on the external surface of the shell (402), and, optionally, each heater of the plurality of heaters (406, 408, 410) is independently controllable to limit ice formation at desired locations.

11. The cryoprobe of claim 8, 9 or 10, further comprising a vacuum sleeve (422) positioned in the inner cavity, wherein an ice formation zone is located at an axial location on the shell (402) between the tip (404) and the vacuum sleeve (422), the at least one heater (406, 408. 410) located on the shell in the ice formation zone.

12. A cryoablation apparatus (100) comprising:
a cryogen delivery apparatus (106) configured to supply a flow of cryogen; and
a cryoprobe (112, 200, 400) operably coupled to the cryogen delivery apparatus (106), the cryoprobe comprising an ice formation limiting device.

13. The cryoablation apparatus of claim 12, wherein the ice formation limiting device comprises a directional insulator (208) positioned radially outward of a cryogen supply (206) in a needle (114) of the cryoprobe (112, 200), and, optionally, the directional insulator (208) comprises an open annulus cross-sectional shape of insulating material and the open portion of the annulus defines a predetermined return path (214) for the cryogen flow.

14. The cryoablation apparatus of claim 12 or 13, wherein the ice formation limiting device comprises a heater (406, 408, 410) positioned on a needle (114) of the cryoprobe (112,400).

15. The cryoablation apparatus of claim 14, wherein the heater (408, 410) is deposited on an external surface of the needle in at least two different conductive inks each having a different resistance.
